# EUROPEAN PATENT APPLICATION

(11) **EP 1 468 696 A1**
(43) Date of publication of application: **20.10.2004**
(21) Application number: 03008989.0
(22) Date of filing: 17.04.2003
(51) Int. Cl.: A61K 45/06, A61K 31/55, A61K 31/7034, A61K 31/19, A61P 17/00

(54) **Combinations of epinastine and antiphlogisitcs as new pharmaceutical compositions for the treatment of skin diseases**

(71) Applicant: Boehringer Ingelheim International GmbH, 55216 Ingelheim am Rhein (DE)
(72) Inventor: The designation of the inventor has not yet been filed

(57) **Abstract**

The present invention relates to a new pharmaceutical composition for the treatment of skin diseases. The composition comprises as pharmacologically active substances an antihistaminic-effective amount of epinastine or a pharmaceutically acceptable salt thereof as well as at least one antiphlogistic compound. The composition also may include pharmaceutically acceptable carriers and excipients.

## Description

The present invention relates to new pharmaceutical compositions for the treatment of skin diseases, comprising as a pharmacologically active substances an antihistaminic-effective amount of epinastine or a pharmaceutically acceptable salt thereof and an antiphlogistic-effective amount of an antiphlogistic compound. The compositions also include pharmaceutically acceptable carriers and excipients.

The invention also relates to the usage of these components for treatment of pruritus (itching) derived from skin disease such as urticaria, eczema, and skin irritation.

Remarkably, the compositions described in the present invention are highly effective in the treatment of skin diseases associated with allergic reactions.

### Background of the invention

In recent years, various factors such as changes in diet and lifestyle, air pollution, increased exposure to environmental chemicals from numerous environmental deterioration, and stress in the social life have caused an increase in the incidence of developing skin diseases associated with allergic reactions such as urticaria, eczema, skin irritation, and dermatitis, as well as skin diseases accompanying itching represented by pruritus, prurigo, psoriasis vulgaris, and the like.

It is emphasized that improvements of surrounding environmental factors such as causative antigen elimination are the most important attempt to start the treatment of these skin diseases, particularly for allergic skin diseases. Nevertheless, as already reviewed, the pathogenic causes vary so widely that they often cannot be specified. For the above reason, combination drugs composed of antihistaminic components are needed frequently for effective treatments of these symptoms including itchy sensation caused from skin diseases.

A tablet formulation composed of phenylephrine hydrochloride, epinastine hydrochloride, isopropamide iodide, glycyrrhizinate dipotassium, lidocaine hydrochloride, and anhydrous caffeine as pharmacological active compounds is disclosed in Example 3 of JPH10-298107A.

This example is a combination drug composed of such as epinastine and glycyrrhizinate dipotassium. The drug has extremely potent suppressive action on airway hypersecretion or cold syndrome therapeutic agent, and is not a treatment for skin disease.

### Objective of the present invention

The present invention intends to provide the compositions for treatment of skin diseases by employing highly effective pharmaceutical compounds for significant improvements on symptoms of skin diseases accompanying itching, particularly urticaria, eczema, skin fit, dermatitis, pruritus, eruption, and psoriasis vulgaris accompanying itchy sensation.

### Description of the invention

The invention relates to the pharmaceutical compositions for the treatment of skin diseases, comprising as pharmacologically active substances an antihistaminic-effective amount of epinsastine or a pharmaceutically acceptable salt thereof as well as an antiphlogistic-effective amount of an antiphlogistic.

Epinastine, (±) 3-amino-9, 13b-dihydro-1H-dibenz [c, f] imidazo [1,5-a] azepine, is a drug possessing H1-antihistaminic property. It primarily has been used to treat allergic reaction of the eyes and the nasal mucosa.

In the composition of the present invention, Epinastine preferably is taken in the form of a salt such as the hydrochloride, hydrobromide, oxalate, nitrate, sulfonate, fumarate, maleate, sulfate, and phosphate. The free base can be taken, too. Preferred is Epinastine-hydrochloride.

The amount of Epinastine or a pharmacologically acceptable salt thereof depends on the application route.

In the case of oral application, the daily dosage in equivalent quantity of Epinastine-hydrochloride for an adult is between 2 and 20 mg, preferably between 5 and 15 mg, and further more preferably between 7.5 and 12.5 mg. Preferably, this amount is given via one or more dosage units.

In the case of topical application, the amount in equivalent quantity of Epinastine hydrochloride is between 1 and 50 mg per 1 g of composition, preferably between 2 and 30 mg per 1 g of composition, and further more preferably between 5 and 15 mg per 1 g of composition.

The pharmaceutical compositions for treatment of skin disease of the present invention include antiphlogistics in addition to epinastine mentioned above.

Antiphlogistics employed to formulate the present invention are preferably selected form the group of glycyrrhizinic acid (glycyrrhizin) and/or a salt thereof, glycyrrhetinic acid and/or a salt and/or derivative thereof, and/or tranexamic acid and/or a salt thereof. The inventive formulation may comprise one ore more of these antiphlogistics. Theses substances can be used as the neutral compounds or as pharmacologically acceptable salts.

Glycyrrhizinic acid, 20beta-carboxy-11-oxo-30-norolean-12-en-3beta-yl-2-O-beta-D-glucopyranuronosyl-alpha-D-glucopyranosid-uronic acid, is a natural triterpenoid saponine and is a drug that shows antiphlogistic efficacy in the treatment of detoxication, viral, allergic reactions and the like. It has glucocorticoid-like properties. Monomolecular Glycyrrhetinic acid, 3beta-hydroxy-11-oxoolean-12-en-30-oid acid and bimolecular glucuronic acid are other representatives of this chemical family with similar pharmacological properties.

Examples of pharmacologically acceptable salts of glycyrrhizinic acid include dipotassium glycyrrhizinate, potassium glycyrrhizinate, monoammonium glycyrrhizinate, di-ammonium glycyrrhizinate, and the like.

Moreover, examples of the derivatives of glycyrrhetinic acid include glyceryl glycyrrhetinate, stearyl glycyrrhetinate, and the like.

Tranexamic acid, trans-4-(aminomethyl)cyclohexanecarboxylic acid, is a drug showing anti-inflammatory effect, hemostatic action, and antiallergic action by preventing plasmin action.

The compositions of the present invention may also include other pharmacologically active substances such as group B vitamins and vitamin-like active substances such as vitamin B₁, vitamin B₆, vitamin B₁₂, niacin, pantothenic acid, biotin, folic acid, orotic acid, lipoic acid, p-aminobenzoic acid, inositol, carnitine, and choline, antioxidant vitamins and antioxidant vitamin-like substances such as vitamin C, vitamin E, vitamin A, ubiquinone, pangamic acid, and flavonoid, sulfur containing amino acid such as cysteine, methionine, aminoethylsulfonic acid, and glutathione, and vitamin D such as ergocalciferol and cholecalciferol - in addition to epinastine and the antiphlogistic mentioned above.

Although the amount of the antiphlogistics in the inventive formulation may vary in dependcy of the type of the antiphlogistic, the applicaiton route etc, the typical amount for oral use given daily to an adult lies in the range from 1 to 2000 mg, and for topical use it lies in the range from 0.1 to 200 mg/g.

Concerning glycyrrhizinic acid and its salt as well as glycyrrhetinic acid the amount for the inventive formulation for oral use given daily to an adult preferably is in the range from 1 to 800 mg, preferably in the range from 2 to 400 mg, more preferably in the range from 15 to 200 mg. For topical use, it lies normally in the range within 200 mg/g, preferably in the range from 0.1 to 50 mg/g, more preferably in the range from 0.2 to 20 mg/g.

Concerning tranexamic acid and its salt the amount for the inventive formulation for oral use given daily to an adult preferably is in the range from 10 to 2000 mg, preferably in the range from 100 to 1000 mg, more preferably in the range from 200 to 750 mg.

For topical use, it lies normally in the range within 200 mg/g, preferably in the range from 1 to 50 mg/g, more preferably in the range from 5 to 20 mg/g.

Pharmaceutical compositions described in the present invention may be orally given once or more times, and may be topically applied once or more times directly onto the affected legion of skin. The dose of epinastine and/or the antiphlogistic may be adjusted in accordance with age, body weight, and manifesting symptoms.

In addition, when the pharmaceutical compositions described in the present invention are orally given, the formulations may include a fast release component comprising epinastine (or epinastine and part of antiphlogistics) and a slow release component comprising part of or all of the antiphlogistic. When other additional active components are added, they may be added to slow release part and/or the fast release part of the formulation in accordance with the pharmacokinetic characteristic of each active component.

These fast release components and slow release components may be part of one single formulation (unit formulations) or they may be formulated separately in at least two independent formulations. Examples of such unit formulations may include multilayer tablets combining fast release layer(s) and slow release layer(s), granules combining fast release granules and slow release granules or capsules filled with the combination of granules, hard capsules filled with a combination of fast release tablet(s) and slow release tablet(s) both in small size, and dry syrup or suspension syrup using microcapsule or microsphere as slow release components.

The pharmaceutical compositions described in the present invention can be used in any oral form such as tablets, granules, fine granules, powders, capsules, caplets, soft capsules, pills, suspensions, emulsions, oral solutions, syrups, dried syrups, chewable forms, forming tablets, drops, and orally disintegrate tablets, and in any topical form such as creams, ointments, gel ointments, suppositories, poultices, tapes, topical solutions, aerosols, lotions, and foams. In addition, the components of the inventive formulations or at least parts of them may also be present in the form of microparticles. Therefore microcapsule, nanocapsules, microspheres, nanospheres, liposomes may be used to includ the aforementioned compositions.

Moreover, the properties of the inventive composition such as stability, release, continuance, disintegration, distinglation, dissolution, concealment of taste, improvement in usage etc. can be regulated by the addition of additives known in the art.

For example the pharmaceutically active substance can be dispensed in separate granules, multi-layer granules, multi-layer tablets or dry coated tablets, tablets of separated granules, microcapsules, etc. Coating preparations such as sugarcoated tablets, film coating tablets, coating granule, foaming pharmaceutical preparation can be used as well as chewable preparations, orally disintegrate tablets, matrix preparations, together with comminutions, solid solutions, etc. Sweetening agents, refrigerants, antioxidants or stabilizing agents, agents adjusting a certain pH-value can be added as well as the viscosity, the osmotic pressure or the salt concentration influencing agents. These methods can also be combined.

Optionally, also the following additives can be added: excipients, bases, binders, disintegrators, lubricants, superplasticizers, coating agents, sugar coating agents, plasticizers, antifoaming agents, polish, foaming agents, antistatic agents, desiccant, moisturizing agents, surfactant, solubilizer, buffer agents, resolvents, solubilizing agents, solvents, diluents, stabilizers, emulsifying agents, suspension, suspending agents, dispersing agents, isotonizing agents, aerosol propellant, adsorbents, reducing agents, antioxidant, backing, wetting agents, wet modifier, filler, extender, adhesives, viscous agent, softeners, pH modifiers, antiseptics, preservatives, sweetening agents, corrigent, refrigerative agents, flavoring agents, perfume, fragrance, coloring matters, and the like. Any of these additives may be used in the regular compositions methods, and do not impose any limitation to such composition methods.

Examples of these additives are explained in the Japanese Pharmaceutical Excipients Directory 2000 (Japan Pharmaceutical Excipients Council edit, Yakuji Nippo. Ltd. issue).

These preparations can be manufactured in the usual manner, i.e. by adding preparation additives to the pharmacologically active substance.

The compositions described in the present invention are explained by examples which follow. However, the present invention of the pharmaceutical compositions is not limited to these examples.

### Examples

### Example 1

### Tablet

The following ingredients were homogeneously mixed. The resulting mixed particles were compressed with a mold to prepare tablets of 250 mg each.

| | |
|---|---|
| Epinastine hydrochloride | 20 g |
| Potassium glycyrrhizinate | 360 g |
| Pyridoxine hydrochloride | 45 g |
| Nicotinamide | 450 g |
| Calcium pantothenate | 60 g |
| Lactose | 481 g |
| Microcrystalline cellulose | 506 g |
| Light anhydrous silicic acid | 14 g |
| Magnesium stearate | 10 g |
| Talc | 4 g |

### Example 2

### Powder

The following ingredients were homogeneously mixed. The resulted mixed particles were divided into portions of 600 mg to prepare powder compositions.

| | |
|---|---|
| Epinastine hydrochloride | 5 g |
| Tranexamic acid | 375 g |
| Corn starch | 238 g |
| Lactose | 270 g |
| Magnesium stearate | 12 g |

### Example 3

### Two layer tablet comprising an A layer and a B layer

The following ingredients of A layer and B layer were processed according a standard method to provide mixed particles, respectively, and the particles were compressed to form two layer tablet at 250 mg(A layer 100 mg, B layer 150 mg) each.

| A layer | |
|---|---|
| Epinastine hydrochloride | 60 g |
| Lactose | 258 g |
| Microcrystalline cellulose | 270 g |
| Light anhydrous silicic acid | 6 g |
| Talc | 3 g |
| Magnesium stearate | 3 g |

| B layer | |
|---|---|
| Monoammonium glycyrrhizinate | 360 g |
| Pyridoxine hydrochloride | 72 g |
| Lactose | 330 g |
| Hydrogenated oil | 84 g |
| Hydroxypropylmethylcellulose 2208 | 45 g |
| Magnesium stearate | 9 g |

### Example 4

### Oral solution

The following ingredients were dissolved in sterile purified water, added with sodium hydrate to adjust at pH 5, and diluted with sterile purified water to get a total volume of 20 L. The resulted solution was transferred in portions of 50 mL into glass bottles to provide oral solutions.

| | |
|---|---|
| Epinastine hydrochloride | 4 g |
| Glycyrrhizinic acid | 40 g |
| Aminoethylsulfonic acid | 80 g |
| Inositol | 20 g |
| Thiamin nitrate | 4 g |
| Riboflavin sodium phosphate | 4 g |
| Pyridoxine hydrochloride | 4 g |
| Carnitine chloride | 40 g |
| Nicotinamide | 10 g |
| Calcium pantothenate | 8 g |
| Citric acid | 50 g |
| Sodium citrate | 10 g |
| Purified sucrose | 2400 g |
| Caramel | 60 g |
| Sodium hydrate | Adequate amount |
| Antiseptics | Adequate amount |
| Flavor | Trace amount |
| Sterile purified water | Adequate amount |

### Example 5

### Syrup

The following ingredients were dissolved in sterile purified water, added with citric acid to adjust a pH of 2.5. Then they were diluted with sterile purified water to prepare syrup at the total volume of 10 L.

| | |
|---|---|
| Epinastine hydrochloride | 20 g |
| Dipotassium glycyrrhizinate | 220 g |
| Pyridoxine hydrochloride | 20 g |
| Riboflavin sodium phosphate | 40 g |
| Panthenol | 60 g |
| Purified sucrose | 4000 g |
| Sodium chloride | 30 g |
| Sodium citrate | 20 g |
| Citric acid | Adequate amount |
| Antiseptics | Adequate amount |
| Flavor | Trace amount |
| Sterile purified water | Adequate amount |

### Example 6

### Sugarcoated tablet

The following ingredients were processed according a standard method to provide mixed particles, and the particle was compressed to form tablets at 240 mg each.

| | |
|---|---|
| Epinastine hydrochloride | 10 g |
| Dipotassium glycyrrhizinate | 200 g |
| L-cysteine | 120 g |
| Ascorbic acid | 100 g |
| Pyridoxine hydrochloride | 50 g |
| Calcium pantothenate | 30 g |
| Riboflavin butyrate | 12 g |
| Lactose | 640 g |
| Corn starch | 406 g |
| Microcrystalline cellulose | 306 g |
| Low substituted hydroxypropylcellulose | 130 g |
| Hydroxypropylcellulose | 90 g |
| Light anhydrous silicic acid | 45 g |
| Talc | 12 g |
| Magnesium stearate | 9 g |

Subsequently, the tablets were transferred into a coating pan, and coated using coating solution. The equal volume mixture of ethyl alcohol contained 5% weight/volume of hydroxypropylmethylcellulose and purified water to increase in weight/volume by 10 mg per one tablet. Next, 2% weight/volume of talc, 2% weight/volume of titanium oxide, 3% weight/volume of calcium carbonate, 1% weight/volume of powdered acacia, and aqueous solution containing 60% weight/volume of purified sucrose were used to coat tablets to give increase in weight/volume by 100 mg per one tablet. Finally, aqueous solution containing 60% weight/volume purified sucrose was used to coat tablets to give an increase in weight/volume by 100 mg per one tablet. Thus sugarcoated tablets were prepared.

### Example 7

### Granules

The following ingredients were prepared as granules according a standard method to prepare mixed particles, and packed to give an amount of 1000 mg per one pack for granules.

| | |
|---|---|
| Epinastine hydrochloride | 10 g |
| Glycyrrhizinic acid | 90 g |
| Thiamin nitrate | 5 g |
| Riboflavin | 5 g |
| Pyridoxine hydrochloride | 6 g |
| Nicotinamide | 30 g |
| Orotic acid | 90 g |
| Hesperidin | 120 g |
| DL-methionine | 100 g |
| Calcium carboxymethylcellulose | 240 g |
| Mannitol | 1500 g |
| Corn starch | 662 g |
| Tartaric acid | 100 g |
| Aspartame | 20 g |
| Acesulfame potassium | 20 g |
| Fragrant materials | 2 g |

### Example 8

### Cream

The following ingredients were processed according a standard method to form a cream of a total weight of 1kg, added with sodium citrate to adjust at pH 5.

| | |
|---|---|
| Epinastine hydrochloride | 10.0 g |
| Glycyrrhetinic acid | 10.0 g |
| Pyridoxine hydrochloride | 1.0 g |
| Medium chain fatty acid triglyceride | 200.0 g |
| Propylene glycol | 150.0 g |
| Glyceryl monostearate | 80.0 g |
| Polyoxyethylene cetyl ether | 40.0 g |
| Diisopropyl adipate | 50.0 g |
| Citric acid | 0.1 g |
| Sodium citrate | Adequate amount |
| Antiseptics | Adequate amount |
| Purified water | Adequate amount |

### Example 9

### Ointment

The following ingredients were processed according a standard method to form a ointment of a total weight of 1kg.

| | |
|---|---|
| Epinastine hydrochloride | 10 g |
| Glycyrrhetinic acid | 10 g |
| Crotamiton | 100 g |
| Lidocaine | 20 g |
| Chlorhexidine hydrochloride | 2 g |
| Paraffin | 40 g |
| Cetanol | 30 g |
| White beeswax | 30 g |
| White petrolatum | Adequate amount |

## Claims

1. A pharmaceutical composition for the treatment of skin diseases comprising as pharmaceutically active ingredients epinastine or a pharmaceutically acceptable salt thereof as well as at least one antiphlogistic compound.

2. The pharmaceutical compositions according to claim 1, **characterized in that** the composition is for oral use and the amount of epinastine or a pharmaceutically acceptable salt thereof daily given to an adult lies in the range from 2 to 20 mg in equivalent quantity to epinastine hydrochloride.

3. The pharmaceutical composition according to claim 1 or 2, **characterized in that** the composition is for topical use and the amount of epinastine or a pharmaceutically acceptable salt thereof lies in the range from 1 to 50 mg in equivalent quantity to epinastine chloride per 1 g of the formulations.

4. The pharmaceutical composition according to any of claims 1 to 3, **characterized in that** the antiphlogistic compound is selected form the group of glycyrrhizinic acid, glycyrrhizin respectively and/or a salt thereof, glycyrrhetinic acid and/or a derivative thereof, and/or tranexamic acid and/or a salt thereof.

5. The pharmaceutical composition according to claim 3, **characterized in that** the derivative of glycyrrhetinic acid is glyceryl glycyrrhetinate and/or stearyl glycyrrhetinate.

6. The pharmaceutical composition according to any of claims 1, 2, 4 or 5, **characterized in that** the composition is for oral use and the daily dose amount of the antiphlogistic compound lies in the range from 1 to 2000 mg.

7. The pharmaceutical composition according to any of claims 1, 3, 4 or 5, **characterized in that** the composition is for topical use and the amount of the antiphlogistic compound lies in the range from 0.1 to 200 mg per 1 g of the formulation.

8. Use of a composition according to any of claims 1 to 6 for the manufacture of a medicament for the treatment of skin diseases.

9. Use according to claim 7, **characterized in that** the skin disease is an allergic caused skin disease.

10. Use of a pharmaceutical composition comprising epinastine or a pharmaceutically acceptable salt thereof and another pharmaceutical composition comprising at least one antiphlogistic compound for the manufacture of a medication for the treatment of skin diseases.

11. Use according to claim 10, **characterized in that** the skin disease is an allergic caused skin disease.

12. Use according to claim 10 or 11, **characterized in that** the epinastine comprising composition is for oral use and the amount of epinastine or a pharmaceutically acceptable salt thereof daily given to an adult lies in the range from 2 to 20 mg in equivalent quantity to epinastine hydrochloride.

13. Use according to any of claims 10 or 11, **characterized in that** the epinastine comprising composition is for topical use and the amount of epinastine or a pharmaceutically acceptable salt thereof lies in the range from 1 to 50 mg in equivalent quantity to epinastine chloride per 1 g of the formulations.

14. Use according to any of claim 10 to 13, **characterized in that** the antiphlogistic compound of the antiphlogistic compound comprising composition is selected from the group of glycyrrhizinic acid, glycyrrhizin respectively and/or a salt thereof, glycyrrhetinic acid and/or is glyceryl glycyrrhetinate glycyrrhetinate and/or a salt thereof and/or stearyl glycyrrhetinate and/or a salt thereof, and/or tranexamic acid and/or a salt thereof.

15. Use according to any of claims 10 to 14, **characterized in that** that the antiphlogistic compound comprising composition is for oral use and the daily dose amount of the antiphlogistic compound lies in the range from 1 to 2000 mg.

16. Use according to any of claims 10 to 14, **characterized in that** that the antiphlogistic compound comprising composition is for topical use and the amount of the antiphlogistic compound lies in the range from 0.1 to 200 mg per 1 g of the formulation.
